# EUROPEAN PATENT APPLICATION

(11) **EP 4 385 423 A1**
(43) Date of publication of application: **19.06.2024**
(21) Application number: 22212782.1
(22) Date of filing: 12.12.2022
(51) Int. Cl.: A61B 8/08, A61B 8/02, G06T 7/00, A61B 8/00

(54) **GENERATING ULTRASOUND MOTION DATA**

(71) Applicant: Koninklijke Philips N.V., 5656 AG Eindhoven (NL)
(72) Inventor: SHULEPOV, Sergei Y., Eindhoven (NL); BARAGONA, Marco, Eindhoven (NL); DE WILD, Nico Maris Adriaan, 5656AG Eindhoven (NL); PHAM, Hoa, Eindhoven (NL); MATTUR, Shashaank Aswatha, Eindhoven (NL)
(74) Representative: Philips Intellectual Property & Standards

(57) **Abstract**

A mechanism for producing ultrasound motion data of a particular region of interest. An ultrasound transducer is controlled to iteratively produce ultrasound image data. The iteratively produced ultrasound image data is processed to track the location of a portion representing a region of interest (which contains the particular region of interest). The tracked location is used to control the production of the ultrasound motion data.

## Description

### FIELD OF THE INVENTION

The present invention relates to the field of ultrasound monitoring of one or more fetuses.

### BACKGROUND OF THE INVENTION

One of the most common approaches for monitoring a fetus or fetuses is through the use of ultrasound-derived data, or simply ultrasound data. There is an increasing interest in the use of an ultrasound system to produce ultrasound motion data responsive to a motion of a region of interest. One example of such ultrasound motion data is a fetal heartrate (FHR) signal which represents the motion (heartbeat(s)) of a fetal heart. Accurate identification of fetal heartrate is particularly important for assessing the state of a fetus and to determine whether intervention is required during labor. Another example of ultrasound motion data is a diaphragm movement signal which represents motion of a diaphragm of a fetus.

One common form of ultrasound motion data is imageless data, also known as non-imaging data. It will be appreciated that the imageless data may, in practice, be presented in a graphical form. Thus, the term imageless data or non-imaging data refers to data that does not contain a representation of the visual appearance (i.e., image) of any anatomical feature or element.

In traditional ultrasound systems for producing imageless data, a clinical expert will place an ultrasound transducer on the abdomen of a pregnant individual, and then manipulate the transducer until it is positioned over a particular region of interest (e.g., the fetal heart). The clinical expert will then adjust a depth setting on the ultrasound transducer until the transducer is correctly monitoring the region of interest at the right depth, i.e., producing ultrasound motion data responsive to a motion of the region of interest.

Traditional ultrasound systems have a number of drawbacks. In particular, optimal placement of the transducer is a challenge, and the expertise of an operating personnel is not always sufficient to match this. It is also recognized that incorrect placement or configuration of the ultrasound transducer (e.g., to set the correct depth) can cause incorrect or low-quality capture of the ultrasound motion data, such as ultrasound motion data that is highly sensitive to other motions apart from the region of interest (e.g., a motion of the pregnant individual or a motion of the pregnant individual's heart).

It is also recognized that placement or positioning of the transducer is likely to vary between different operating personnel. This means that there is inconsistency in the capture and subsequent processing (e.g., measurements) of ultrasound data.

There is a therefore a desire to provide an ultrasound system that overcomes these problems of positioning and configuring the ultrasound transducer. It would be even more advantageous if this goal could be achieved in an energy efficient manner.

### SUMMARY OF THE INVENTION

The invention is defined by the independent claims. The dependent claims define advantageous embodiments.

According to examples in accordance with an aspect of the invention, there is provided an ultrasound monitor for performing ultrasound monitoring of one or more fetuses.

The ultrasound monitor comprises: an interface for communicatively coupling with an ultrasound transducer; and a processor configured to be operable in a multi-data mode, when the ultrasound transducer is communicatively coupled to the interface, in which the processor: controls the ultrasound transducer to iteratively produce ultrasound image data of the one or more fetuses; processes the iteratively produced ultrasound image data to continually track the location of a first portion of the ultrasound image data that represents a first region of interest of the one or more fetuses; and uses the tracked location of the first portion to control the ultrasound transducer to iteratively produce ultrasound motion data, that represents a smaller region of the one or more fetuses than the ultrasound image data, that changes responsive to a motion within a second region of interest contained within the first region of interest.

The present disclosure proposes to track the location of a first portion within ultrasound image data (e.g., B-mode image data). The first portion represents a first region of interest within the fetus(es). The tracked location is used to control the production of ultrasound motion data representing a second region of interest. The second region of interest represents a region smaller than that captured in or represented by the ultrasound image data. The second region of interest is entirely contained within the first region of interest.

The proposed approach facilitates more accurate, robust and consistent production of ultrasound motion data of a particular region of interest.

In preferred examples, the ultrasound motion data does not contain a representation of the anatomical appearance of the second region of interest. Thus, the ultrasound motion data may be imageless data or non-imaging data.

In some examples, the processor is configured to, when operating in the multi-data mode: for a first iteration of producing the ultrasound motion data, process the first portion of the most recently produced ultrasound image data to identify the location of the second region of interest with respect to the ultrasound transducer; and use the identified location of the second region of interest to control the ultrasound transducer to iteratively produce the ultrasound motion data.

This technique proposes to perform continuous tracking of anatomical features of interest. This will speed-up identification of the second region of interest and be more power efficiency, as tracking will allow for a smaller second ROI analysis. Tracking a larger region of interest is less resource-intensive then tracking a smaller region of interest.

As compared to starting a new search for the second region of interest, continuous tracking achieves better locating with less processing resource and/or power expense.

In some examples, the processor is configured to, when operating in the multi-data mode: process the iteratively produced ultrasound image data and/or the iteratively produced ultrasound motion data to predict whether or not the second region of interest has moved by more than a predetermined amount with respect to the ultrasound transducer; and responsive to determining that the second region of interest has moved by more than the predetermined amount, process the first portion of the most recently produced ultrasound image data to update the identified location of the second region of interest with respect to the ultrasound transducer.

The processor may be configured to, when operating in the multi-data mode, responsive to determining that the second region of interest has not moved by more than the predetermined amount, maintain the location of the second region of interest with respect to the ultrasound transducer.

These approaches reduce a power consumption, as there is reduced processing of the ultrasound image (or the first portion) in order to maintain accurate generation of the ultrasound motion data. In particular, the location from which ultrasound motion data is acquired is only changed if the second region has moved by more than a predetermined amount (i.e., such that the current location is no longer accurately representative of the location of the second region of interest).

Preferably, the second region of interest is smaller than the first region of interest.

The ultrasound motion data may comprise M-mode ultrasound data and/or Doppler-mode ultrasound data. In some examples, the ultrasound motion data is pulsed-wave Doppler ultrasound data.

The first ultrasound image data and/or the second ultrasound image data may comprise B-mode ultrasound image data.

In some examples, the processor is further configured to, when operating in the multi-data mode, control the ultrasound transducer to iteratively produce color or power Doppler-mode ultrasound data of the one or more fetuses.

In this way, the processor may operate in a mode in which ultrasound image data, ultrasound motion data and color or power Doppler-mode ultrasound data is continually produced.

The processor may be configured to process the iteratively produced ultrasound image data and the iteratively produced color or power Doppler-mode ultrasound data to continually track the location of a first portion of the ultrasound image data that represents a first region of interest of the one or more fetuses.

The processor may be configured, when operating in the multi-data mode to process the ultrasound motion data to produce a measurement for each of one or more clinical parameters of the region of interest and/or generate a time-varying signal that changes responsive to changes in the ultrasound motion data between each iterative production of the ultrasound motion data.

There is also proposed an ultrasound system comprising: the ultrasound monitor previously described; and the ultrasound transducer communicatively coupled to the interface of the ultrasound monitor.

There is also proposed a computer-implemented method for controlling an ultrasound transducer for performing ultrasound monitoring of one or more fetuses. The computer-implemented method comprises: controlling the ultrasound transducer to iteratively produce ultrasound image data of the one or more fetuses; processing the iteratively produced ultrasound image data to continually track the location of a first portion of the ultrasound image data that represents a first region of interest of the one or more fetuses; and using the tracked location of the first portion to control the ultrasound transducer to iteratively produce ultrasound motion data, that represents a smaller region of the one or more fetuses than the ultrasound image data, that changes responsive to a motion within a second region of interest contained within the first region of interest.

There is also proposed computer program product comprising computer program code which, when executed on a processor communicatively coupled to the ultrasound transducer, cause the processor to perform all of the steps of the herein described method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates an ultrasound system in which embodiments can be employed;
Fig. 2 illustrates an ultrasound transducer;
Fig. 3 illustrates example ultrasound data;
Fig. 4 illustrates a pulsed-wave Doppler spectrum;
Fig. 5 illustrates another pulsed-wave Doppler spectrum; and
Fig. 6 is a flowchart illustrating a method according to an embodiment.

### DETAILED DESCRIPTION OF THE EMBODIMENTS

The invention will be described with reference to the Figures.

It should be understood that the detailed description and specific examples, while indicating exemplary embodiments of the systems and methods, are intended for purposes of illustration only and are not intended to limit the scope of the invention. These and other features, aspects, and advantages of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for producing ultrasound motion data of a particular region of interest. An ultrasound transducer is controlled to iteratively produce ultrasound image data. The iteratively produced ultrasound image data is processed to track the location of a portion representing a region of interest (which contains the particular region of interest). The tracked location is used to control the production of the ultrasound motion data.

Embodiments are based on the realization that tracking a location of a part of image data representing a region of interest (containing a desired region of interest) is less resource-intensive then reprocessing the entire image data to identify a portion representing a desired region of interest. This technique can thereby be used to reduce the power required to produce and monitor ultrasound motion data for a desired region of interest.

Proposed approaches can be employed in any environment in which ultrasound monitoring of one or more fetuses is performed, e.g., in a clinical environment or a home environment.

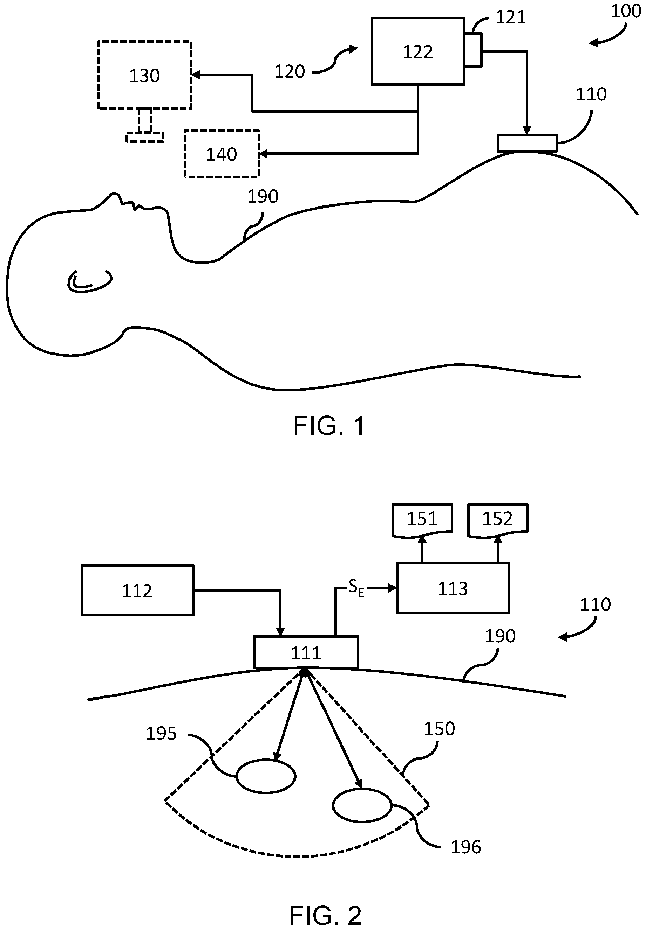

Fig. 1 illustrates an ultrasound system 100 for performing ultrasound monitoring of one or more fetuses (carried by a subject 190). The ultrasound system 100 comprises an ultrasound transducer 110 and an ultrasound monitor 120 according to a proposed embodiment.

The ultrasound system 100 may comprise a user interface 130 and/or a further processor 140. In other examples, these are absent or form separate elements able to communicate with the ultrasound system 100.

The ultrasound transducer 110 is configured to produce ultrasound data of an investigated region. Typically, an ultrasound transducer 110 is configured to transmit ultrasound waves into a subject 190 and receive echo information. The echo information can then be processed by the ultrasound transducer to generate ultrasound data about the investigated region, i.e., the region into which ultrasound waves were emitted. Examples of ultrasound data include ultrasound image data (e.g., B-mode image data) and ultrasound motion data (e.g., data derived from Doppler-based measurement techniques). The ultrasound motion data may be imageless data or non-imaging data, in that it does not contain a representation of the anatomical appearance of an investigated region.

Fig. 2 illustrates an example ultrasound transducer 110. The transducer comprises one or more transducers 111 (e.g., a plurality of transducers), a beam processor 112 and a transducer processor 113. Examples of transducers are well known, and include CMUT (capacitive micromachined ultrasonic transducer) transducers; piezoelectric transducers, formed of materials such as PZT (lead zirconate titanate) or PVDF (polyvinylidene fluoride); or any other suitable transducer technology.

The one or more transducers 111 are configured to transmit ultrasound waves into the subject 190 responsive to the beam processor 112. Thus, the beam processor 112 controls the transmission of ultrasound waves.

The one or more transducers 111 are also configured to receive echo information (being echoes of the transmitted ultrasound waves from objects 195, 196 in the subject 190) and generate an echo signal S_{E} responsive thereto. The transducer processor 113 is configured to process the echo signal to produce the ultrasound data. As previously explained, examples of ultrasound data include ultrasound image data 151 or ultrasound motion data 152.

The beam processor 112 may also control the generation of the echo signal S_{E} by the one or more transducers 111.

Of course, the ultrasound transducer 110 may comprise any necessary circuitry or elements for performing ultrasound monitoring/analysis of a subject. For instance, the beam processor 112 may include beamforming arrangements, beam steering arrangements, drive electronics and/or signal processing circuits (e.g., comprising filtering circuits, sampling circuits, ADCs (analog to digital converters), DACs (digital to analog converters), and so on). Similarly, the transducer processor 113 may comprise any circuitry necessary for processing an echo signal, e.g., filtering circuits, sampling circuits, ADCs, DACs, and so on.

The detailed structure and operation of a suitable ultrasound transducer is well known in the art, and is not explained in detail for the sake of conciseness.

Referring back to Fig. 1, the present disclosure provides an ultrasound monitor 120 that is configured to control the operation of the ultrasound transducer 110. Whilst illustrated as a separate module, for the purposes of illustrative clarity, the ultrasound monitor 120 may in practice form a part of the ultrasound transducer (e.g., a processor of the ultrasound transducer). However, this is not essential.

The ultrasound monitor 120 comprises an interface 121 for communicatively coupling with the ultrasound transducer 110. The ultrasound monitor also comprises a processor 122 configured to control the operation of the ultrasound transducer 110 when it is communicatively coupled to the interface 121. Thus, the processor 122 controls the ultrasound transducer via the interface 121.

The processor is configured to be operable in at least a multi-data mode. The processor may be operable in other modes, which are not described in detail in this disclosure.

When operating in the multi-data mode, the processor is configured to control the ultrasound transducer to iteratively produce ultrasound image data of the one or more fetuses. Thus, ultrasound image data is repeatedly generated and/or updated. In this way, the ultrasound transducer is controlled to repeatedly: transmit ultrasound waves, receive echo information; generate an echo signal responsive thereto; and produce ultrasound image data responsive to the echo signal.

The ultrasound image data contains a representation of the visual appearance of the investigated region, i.e., the region that receives and reflects ultrasound waves emitted by the one of more transducers. Approaches for producing ultrasound image data are well established, and have been previously described.

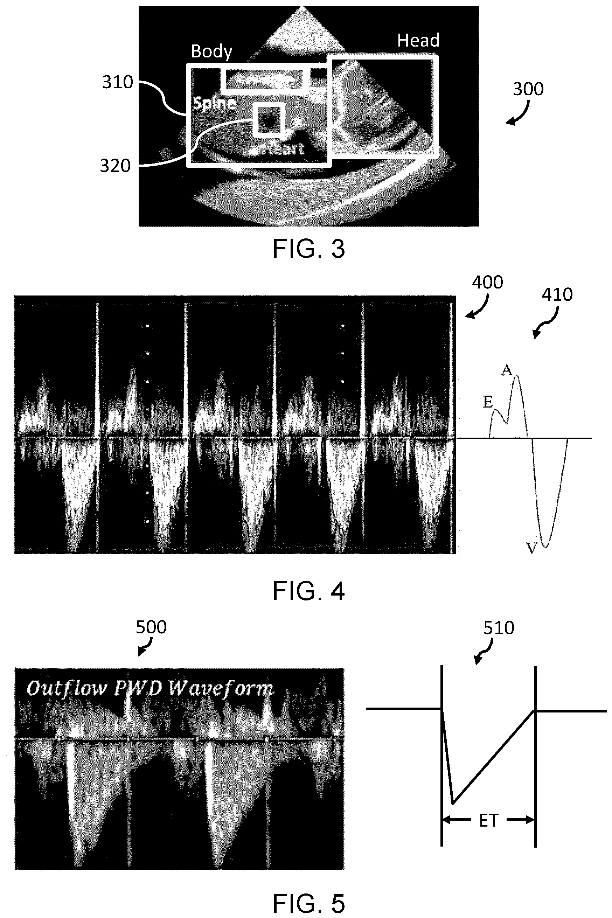

Fig. 3 illustrates example ultrasound data in the form of an ultrasound image 300.

The processor 122 is also configured, when operating in the multi-data mode, to process the iteratively produced ultrasound image data to continually track the location of a first portion of the ultrasound image data that represents a first region of interest of the one or more fetuses.

Approaches for determining the location of a first portion of ultrasound image data representing a first region of interest are well established in the art. The first portion is smaller than the size of the ultrasound image data. A number of suitable examples are provided later in this disclosure.

With reference to Fig. 3, example portions of the ultrasound image data representing different regions of interest are illustrated, in which boxes or bounding boxes illustrate the bounds of the portion of the ultrasound image data. Fig. 3 is used to demonstrate how different portions of ultrasound image data are able to represent different potential regions of interest of the one or more fetuses.

The first region of interest (ROI) may, for example, be one of the one or more fetuses or the body of a fetus. Preferably, the first region of interest is a region that is or contains a second region that undergoes a periodic motion, such as a fetal heart or a fetal diaphragm.

The processor is also configured, when operating in the multi-data mode, to use the tracked location of the first portion to control the ultrasound transducer to iteratively produce ultrasound motion data, that represents a smaller region of the one or more fetuses than the ultrasound image data, that changes responsive to a motion within a second region of interest contained within the first region of interest. The second region of interest is a region of interest of the one or more fetuses.

The ultrasound motion data may be representative of the motion at only the second region of interest (i.e., and not other regions outside the second region of interest).

Thus, there is introduced the concept of a second region of interest contained within the first region of interest. In some examples, the second region of interest is a sub-region within the first region of interest (i.e., a region smaller than but entirely contained by the first region).

With reference to Fig. 3, according to one example, the first region (represented by a first portion 310) may identify a larger region of interest than the second region (representing by a second, smaller portion 320 entirely contained within the first region). In this illustrated example, the first region of interest is a body or torso of the fetus, and the second region of interest is the fetal heart.

In other examples, the first and second regions are the same.

The second region of interest represents a desired or targeted region of the fetus(es), e.g., a desired or targeted anatomy, for which ultrasound motion data is desired. The region of interest may, for instance, be represented by a particular anatomical element or feature (such as the fetal heart or fetal diaphragm). Thus, the second region of interest may bound or encapsulate a desired anatomical feature, element or structure.

Preferably, the second region of interest is a region (e.g., an anatomical feature, element or structure) that undergoes a periodic motion, such as a fetal heart or a fetal diaphragm. In particularly preferable examples, the second region of interest is preferably a region of the fetus(es) for which movement represents movement resulting from a vital sign or other clinical parameter, such as a heartbeat, respiratory rate or the like. In particular, the second region of interest is preferably a region for which a movement is representative of a vital sign or clinical parameter (i.e., a parameter used to monitor the condition of the fetus). Examples include a heart, umbilical cord, lungs, or potentially smaller or more specific aspects of such anatomical structures - such as the mitral-aortic junction, a specific heart chamber (e.g., the left atrium) and so on.

Embodiments are particularly advantageous when the second region of interest is a fetal heart or a portion thereof, as this is an important structure to monitor during the course of pregnancy and/or birth.

The ultrasound motion data may, for instance, comprise one or more of the following and/or data derived from one or more of the following: M-mode ultrasound data; Doppler-mode ultrasound data; and/or pulsed-wave Doppler ultrasound data.

An example of ultrasound motion data a vital sign measurement, i.e., ultrasound motion data that changes responsive to a change in a vital sign of the one or more fetuses. One example of a vital sign measurement is a heartrate measurement, such as a fetal heartrate measurement. Another example is a diaphragm movement measurement, such as a fetal respiratory rate.

Approaches for using an ultrasound transducer to produce ultrasound motion data responsive to movement are well known, and include Doppler-based imaging (which employs the principle of Doppler shift to generate imageless data) such as pulsed-wave Doppler imaging. Typically, such approaches comprise monitoring a position along a particular scanline from a transducer of the ultrasound transducer to monitor a Doppler shift at said position.

M-mode ultrasound data; Doppler-mode ultrasound data; and/or pulsed-wave Doppler (PWD) ultrasound data produced for the location of the heart and/or a particular part of the heart may represent a heartrate measurement. In particular, a PWD spectrum can represent a motion and/or electrical activity of the heart.

In an example, the ultrasound motion data comprises (second) ultrasound image data representing a visual appearance of a smaller region of interest than the ultrasound image data. This approach can allow, for instance, for higher-resolution imaging of a desired region of interest or reduced processing or power requirements to produce an image that still contains the more important region of interest.

In particular, when producing the motion ultrasound data, the ultrasound transducer is controlled or configured to monitor and target a specific region of interest with respect to the ultrasound transducer. This can be achieved by setting the depth/distance and the angle of the investigated/scanned area from the transducer(s) of the ultrasound transducer. The ultrasound transducer may define a scanline that intersects the region of interest and is monitored for changes in intensity, and therefore implied movement, of the region of interest.

There are various techniques that could be exploited to make use of the tracked location of the first portion of the ultrasound image data in order to improve the accuracy of the iteratively produced ultrasound motion data. More particularly, these techniques improve the accuracy of defining a volume of the fetus(es) to be scanned when generating the ultrasound motion data, e.g., defining a depth/distance and the angle. A number of these techniques are hereafter described.

The first and second techniques described below can be used if the first region of interest is larger than the second region of interest. For instance, the first region of interest may represent a body of a fetus and the second region of interest may represent a heart/lungs of a fetus.

The third technique described below can be used if the first region of interest and the second region of interest are the same. For instance, the first and second region of interest may both represent a heart/lungs of a fetus. Thus, in this technique, the first portion represents both the first and second regions of interest.

The first technique makes use of the identified location of the first portion (for the first region) to increase an ease of identifying a location of a second first portion (for the second region).

This technique also makes use of the knowledge that the spatial relationship between any given area/region of image data and the transducer used to produce the image data may be known or determinable. More simply, it is possible to identify a distance and angle that an element, represented by a particular location within an ultrasound image, makes with the transducer that produces the ultrasound image by mapping the particular location in the image to a location in space.

This means that identification of the relative location of a second first portion (representing the second region of interest) within an image facilitates identification a location of a second region (known to be contained by the first region) with respect to the transducer.

In particular, information on the second portion (e.g. position, orientation) can be used to define a sampling volume for the generation of ultrasound motion data.

In particular, if the first region of interest is larger than the second region of interest, then the processor may be configured to process the first portion of the ultrasound image data (for the first region) to identify a second portion of the ultrasound image data representing the second region of interest. By only processing this first portion of the ultrasound image data, the amount of data that needs to be processed to identify the second region of interest is significantly reduced.

The location of the second portion (with respect to the image data) can then be used to identify the location of the second region of interest with respect to the ultrasound transducer, e.g., using a mapping or similar approach - as the correspondence between locations in image data and corresponding real-space locations with respect to the transducer is known. This identified location can then be used to control the ultrasound transducer to monitor the second region of interest in the subject to produce the ultrasound motion data.

This procedure of identifying the location of the second region of interest can be performed, for instance, for the first iteration of producing the ultrasound motion data, after which the determined location of the second region of interest is reused. In some examples, the ultrasound motion data is not generated until the ultrasound image data is available and/or has been processed to identify the location of the first portion of the first region of interest.

In some instances, the procedure of identifying the location of the second region of interest is repeated - e.g., after a certain period of time, or number of instances of ultrasound motion data have been generated, and/or responsive to a user override. Another possible trigger for repeating the identification of the location of the second region of interest is responsive to determining that the second region of interest has moved by more than a predetermined amount.

Determining whether the second region of interest has moved by more than a predetermined amount may be performed by processing the iteratively produced ultrasound image data and/or the iteratively produced ultrasound motion data to predict whether or not the second region of interest has moved by more than the predetermined amount with respect to the ultrasound transducer.

This step may be performed, for instance, by monitoring the tracked location of the first portion representing the first region of interest. If the first portion has moved by more than the predetermined amount, then this indicates that the second region of interest (contained within the first region of interest represented by the first portion) has also moved by the predetermined amount.

Processing the ultrasound motion data to determine whether or not the second region of interest has moved by more than a predetermined amount may comprise processing the ultrasound motion data to determine whether or not the ultrasound motion data meets at least one of one or more predetermined criteria. The one or more criteria may be criteria that indicate whether or not the second region of interest has moved. Thus, each criterion should be a criterion that is at least partially responsive to a change in location of the second region of interest with respect to the ultrasound transducer.

Various criteria can indicate or respond to a change in position of the second region of interest. A non-exhaustive number of examples are hereafter provided.

As one example, a first criterion may be an amplitude (e.g., an average or peak amplitude) of the ultrasound motion data falling below a predetermined threshold amplitude. If the value(s) of the ultrasound motion data diminishes significantly, this is a sign of an anomalous reading and therefore a sign that the region of interest has moved with respect to the ultrasound transducer. In particular, it may be due to casualty or a change in the localized position of the region of interest due to maternal or fetal movement, either of which would call for intervention.

In an example, a second criterion may be a disruption to a periodicity in the ultrasound motion data. If a periodicity of the ultrasound motion data undergoes a sudden shift or change, then this is indicative that the region of interest has moved or been repositioned.

By way of example only, the second criterion may be that there is a change of more than X in the periodicity of the ultrasound motion data within a time window of Y seconds, where X and Y are predetermined. As a working example, if the second region of interest is a fetal heart and the ultrasound motion data is a value representing a fetal heartrate, the value of X may be between 10 and 30 bpm (e.g., 20 bpm) and the value of Y may be between 1 and 3 seconds (e.g., 2 seconds).

A third criterion may be the presence of one or more anomalous variations in the ultrasound motion data. Anomalous variations may indicate the presence of another pulsating or moving component (i.e., other than the region of interest) influencing the ultrasound motion data, indicating that the region of interest has moved.

To determine whether the third criterion has been met, the ultrasound motion data may be processed using an appropriate technique, such as a stochastic signal analysis technique, to identify the presence or absence of any anomalous variations in the ultrasound motion data. A suitable examples of a stochastic signal analysis technique is disclosed by Bechet, P., R. Mitran, and M. Munteanu. "A noncontact method based on multiple signal classification algorithm to reduce the measurement time for accurately heart rate detection." Review of Scientific Instruments 84.8 (2013): 084707.

The above-identified example criteria are particularly advantageous when the ultrasound motion data is imageless data.

As previously explained, the generated indicator may indicate whether or not one or more of these criteria have been met (i.e., whether or not the region of interest has moved with respect to the ultrasound transducer). The generated indicator may indicate that the region of interest has moved if any of these criteria are met. Of course, it is not essential that all of the above-mentioned criteria be monitored in the process 380, rather only a subset (i.e., not all) may be monitored.

The second technique makes use of the identified location of the first portion to identify a movement of the first region of interest (and therefore of the second region of interest).

In this approach, if the tracked location of the first portion moves by more than a predetermined movement (e.g., over a certain time period or number of times ultrasound image has been produced), then this may trigger the (re)identification of the location of the second region of interest, e.g., by processing the overall ultrasound image data or just the portion of the ultrasound image data bound by the first portion (for the first region).

The third technique makes use of the identified location of the first portion to directly track the movement of the second region of interest with respect to the ultrasound transducer systm

In particular, if the first region of interest is the same as the second region of interest, then the tracked location of the first portion can be directed used to identify the location of the second region of interest with respect to the ultrasound transducer (i.e., without the need to explicitly identify the location of the second portion). This can be used to update the control of the ultrasound transducer to continue monitoring the second region of interest.

Of course, any suitable combination of the proposed techniques can be used to advantage. For instance, the first and second techniques can be readily combined.

Regardless of which of the techniques (or combinations thereof) are used, it will be apparent that the proposed approach allows for improved identification of the location of a specific region of interest with respect to the transducer, which allows for directed and specific generation of ultrasound motion data for that region of interest. In this way, the ultrasound motion data is produced within a well-defined region of interest. Recorded signals (i.e., ultrasound motion data) therefore have much better signal-to-noise ratios, enabling a better quality of monitoring and/or future processing.

Furthermore, the signals (i.e., ultrasound motion data) are less contaminated by other sources, which would enable assessment of not only simple features of any vital signs in the region of interest, such as heart rate, but also more sophisticated features such as fiducial points on the traces. This opens a perspective of the early antenatal diagnostics of low sensitivity/dependency on the user/operator.

Although above-described examples only provide a single instance of iteratively updated ultrasound motion data, it will be apparent that (in some embodiments) there may be a plurality of second regions of interest for which a corresponding plurality of instances of iteratively updated ultrasound motion data are produced.

In above-described embodiments, when the processor is operating in the multi-data mode, the ultrasound transducer is controlled by the processor to produce ultrasound image data and ultrasound motion data. Thus, the previously described multi-data mode is effectively a duplex mode of operation.

However, in some embodiments, the multi-data mode may be configured such that, when the processor is operating in the multi-mode system, the processor is further configured to control the ultrasound transducer to iteratively produce color or power Doppler-mode ultrasound data of the one or more fetuses.

In this way, the multi-data mode may effectively be a triplex mode.

The generation of color and/or power Doppler-mode ultrasound data (as well as the ultrasound image and ultrasound motion data) is advantageous in that it facilitates improved identification of the anatomical features. More particularly, spatiotemporal correlation applied to the data produced in this multi-data mode will allow for an improved tracking of the first portion in the ultrasound image data.

Thus, the processor is configured to process the iteratively produced ultrasound image data and the iteratively produced color or power Doppler-mode ultrasound data to continually track the location of a first portion of the ultrasound image data that represents a first region of interest of the one or more fetuses.

In particular, the iteratively produced color or power Doppler-mode ultrasound data may act as a further input to a process that tracks the location of the first portion of the ultrasound image data. It will be appreciated that the spatial relationship between the Doppler-mode ultrasound data and the ultrasound image data is readily determinable.

Generating color or power Doppler-mode ultrasound data is also advantageous for improving future processing of all ultrasound data, e.g., for improving the classification of the imaging plane orientation (e.g., echocardiographic views). An improved fetal heart evaluation has also been demonstrated in L. YEO and R. ROMERO, Color and power Doppler combined with Fetal Intelligent Navigation Echocardiography (FINE) to evaluate the fetal heart, Ultrasound Obstet Gynecol 2017; 50: 476-491, indicating that the use of such data with other ultrasound data can improve the identification of valuable clinical information.

The (iteratively produced) ultrasound image data and/or ultrasound motion data may be further processed to derive or produce additional data, e.g., additional vital sign information or details. The skilled person would readily appreciate that the precise processing of the ultrasound image data and/or ultrasound motion data and/or the nature of the additional data may depend upon the content of the second region of interest (i.e., the investigated anatomy).

This further processing may be performed by the processor or a/the further processor 140, e.g., processing the data generated by the ultrasound transducer as controlled by the processor.

More particularly, the processor and/or a further processor may be configured to process the ultrasound motion data to produce a measurement for each of one or more clinical parameters of the region of interest.

In an example, the processor and/or a further processor may be configured to generate a time-varying signal that changes responsive to changes in the ultrasound motion data between each iterative production of the ultrasound motion data. This can effectively represent a pattern of movement of the second region of interest.

In an example, the second region of interest is the mitral-aortic junction, and the ultrasound motion data is pulsed-wave Doppler (PWD) ultrasound data, such as a PWD spectrum or flow velocity waveform derivable therefrom, produced for the mitral-aortic junction.

Fig. 4 illustrates an example PWD spectrum 400 (i.e., example ultrasound motion data) produced using a proposed approach.

This approach allows the morphologies or fiducial points of the diastolic and systolic functions to be recognized in the PWD spectrum. Thus, in this example, it is possible to process the ultrasound motion data alone to derive additional data about one or more vital signs.

More particularly, it is possible to process or trace the PWD spectrum 400 to produce a flow velocity waveform 410. The systolic phase will produce a single peak in the flow velocity waveform, caused by blood flow through the aortic valve. The diastolic phase forms a first peak E determined by the passive, "early" filling of the left ventricle through the mitral valve, and a second peak A due to the active atrial contraction. Overall, the E+A-wave represents the mitral inflow, whereas the V-wave represents the aortic outflow.

Typical quantitative cardiac performance indexes can be extracted based on the positive and negative envelopes, such as the heart rate, the E/A ratio, the mechanical AV conduction time interval and more. These time indexes are useful indicators of fetal wellbeing and can also be used in early in diagnostics of specific diseases.

As another example, the second region of interest is a specific heart chamber or other heart feature such as a valve, of a fetus. In this way, the function of a particular chamber represented in the ultrasound image data can be individually assessed. Ultrasound motion data could, for instance, be produced for each heart chamber of the fetus - such that there is a plurality of second regions of interest for which ultrasound motion data is generated.

By way of example, the overall heart rate can be computed based on the PWD waveforms from individual chambers. Other clinical indices could be estimated by combining waveforms from different chambers or features using a specific duty-cycle pre-set, based on the programmed anatomical feature detection.

Fig. 5 illustrates one embodiment of this example. The PWD spectrum 500 ("Outflow PWD waveform") is generated for the pulmonary valve (which acts as the second region of interest). It is possible to process or trace the PWD spectrum 500 to produce a flow waveform 510. This can, in turn, be processed to identify the movement of the pulmonary value, e.g., to determine the ejection time ET between opening and closing of the valve. The ejection time is one example of an important clinical parameter for assessing the condition of a fetus.

In an example, the second region of interest may be the umbilical cord. In this example, the color or power Doppler-mode data is also generated (of the umbilical cord).

Umbilical arterial (UA) Doppler assessment is commonly used in surveillance of fetal well-being in the third trimester of pregnancy. Abnormal umbilical artery Doppler is a marker of placental insufficiency and consequent intrauterine growth restriction (IUGR) or suspected pre-eclampsia. Umbilical artery Doppler assessment has been shown to reduce perinatal mortality and morbidity in high-risk obstetric situations.

It will therefore be apparent that additional information about the umbilical would prove clinically useful for aiding in the assessment of the monitoring of fetal well-being.

By way of example, the ultrasound motion data can be processed to predict or determine one or more of the following: umbilical arterial S/D ratio (SDR): systolic velocity / diastolic velocity; pulsatility index (PI) (Gosling index): (PSV - EDV) / TAV; resistive index (RI) (Pourcelot index): (PSV - EDV) / PSV; PSV: peak systolic velocity; EDV: end-diastolic velocity; and/or TAV: time-averaged velocity.

If present, the user interface 130 and/or further processor 140 may be configured to receive at least the ultrasound motion data and/or data derived therefrom. For instance, the processor may provide motion output data to the user interface 130 and/or further processor 140. The motion output data includes the ultrasound motion data and/or data derived from the motion ultrasound data. Preferably, the motion output data does not include data directly derived from the ultrasound image data.

The user interface 130, if present, may provide a visual representation of the ultrasound motion data and/or data derived therefrom (e.g., the motion output data). Correspondingly, the ultrasound processor 120 may be configured to control a user interface 130 to provide a visual representation of the ultrasound motion data and/or data derived therefrom.

Similarly, the further processor 140, if present, may perform further processing on the ultrasound motion data and/or data derived therefrom, e.g., to analyze or generate alerts responsive to the ultrasound motion data.

In a desirable mode of operation, the one or more transducers of the ultrasound transducer are applied at a particular location on the subject to be investigated. Ultrasound image data may be produced by the transducer and processed by the processor to identify a location of a first portion of the ultrasound image data representing a first region of interest. If the ultrasound image data does not contain the first portion (i.e., there is no representation of the first region of interest), then the user may be instructed/guided to move the transducer(s) until the first portion is identified. When the first portion is identified, then the processor may enter the multi-data mode described above.

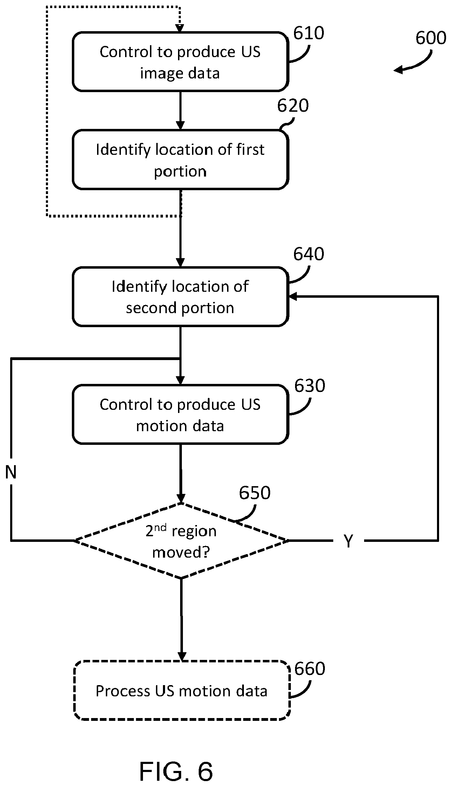

Fig. 6 is a flow-chart illustrating a computer-implemented method 600 performed by a processor according to an embodiment. The computer-implemented method 600 represents the steps taken when the processor is operating in a multi-data mode.

The method 600 comprises a step 610 of controlling the ultrasound transducer to iteratively produce ultrasound image data of the one or more fetuses.

The method 600 also comprises a step 620 of processing the iteratively produced ultrasound image data to continually track the location of a first portion of the ultrasound image data that represents a first region of interest of the one or more fetuses.

The iterative repetition of step 610 is illustrated by a dotted line connecting steps 620 to 610.

The method 600 also comprises a step 630 of controlling the ultrasound transducer to iteratively produce ultrasound motion data. Step 630 is performed using the using the tracked location of the first portion. The ultrasound motion data represents a smaller region of the one or more fetuses than the ultrasound image data. The ultrasound motion data changes responsive to a motion within a second region of interest contained within the first region of interest. The second region of interest is a region of interest of the one or more fetuses.

In some examples, the method 600 comprises a step 640 of identifying a location of a second portion of the image (contained within the first portion), the second portion representing the second region of interest in the (most recently produced) ultrasound image data.

The identified location of the second portion of the image can be used to identify the location of the second region of interest with respect to the transducer. This identified location can be used to control the transducer (e.g., set a depth and/or direction of a scan line) in order to generate the ultrasound motion data of the second region of interest.

Step 640 may comprise processing only the identified first portion of the ultrasound image data to identify the location of the second portion. Alternatively, step 640 may comprise processing the entire ultrasound image data to identify the location of the second portion.

Step 640 may be performed for at least a first iteration of performing step 630. Step 640 may be repeated if, for instance, it is determined that the second region has moved by more than a predetermined amount in a determining step 650. Approaches for performing step 650 have been previously described. There may be other triggers for repeating step 640, e.g., after a certain period of time has elapsed or if more than a predetermined number of instances of ultrasound motion data have been produced.

Step 640 may be omitted if, for instance, the first and second regions of interest are the same. In this scenario, the tracked location of the first portion can be directly used to control the ultrasound transducer to iteratively produce the ultrasound motion data.

The method 600 optionally comprises an ultrasound (US) motion data processing step 660. The US motion data processing step 660 may comprise processing the ultrasound motion data to produce a measurement for each of one or more clinical parameters of the region of interest. Additionally or alternatively, step 660 comprises generating a time-varying signal that changes responsive to changes in the ultrasound motion data between each iterative production of the ultrasound motion data.

Embodiments involve the processing of ultrasound image data, or a portion thereof, in order to identify the location of a first portion representing a first region of interest or a second portion representing a second region of interest.

Typically, identification of a portion includes processing the ultrasound image data to identify the location of a plurality of bounding boxes ("boxes") or potential sets of one or more landmarks ("sets"), each box/set representing a potential region of interest, within the image data. Of course, the identified plurality of boxes/sets may undergo non-maximum suppression (or a similar technique) as a filtering technique.

A bounding box may, for instance, have a rectangular/cuboidal shape or may otherwise comprise a segmented shape representing the region of interest. A landmark may be a single point or location representing a particular feature or element within a region of interest.

One or more of the boxes/sets may then be selected (e.g., based on a confidence level for each portion) as representative of the region of interest. In particular, a first box/set may be selected as indicative of the location of a portion if a confidence level for that box/set exceeds some predetermined threshold or the confidence level is within the top B number or percentage of confidence levels, where B is predetermined.

The mapping between any given area/region of image data and the spatial relationship between the object represented by that area and the transducer used to produce the image data may be known in advance or determinable. In other words, for any position in ultrasound image data, the distance and direction of the absolute location (in real space) represented by that position from the ultrasound transducer can be readily determined, e.g., using well established approaches. This facilitates mapping from the location of the box/set to a location in space.

One approach to processing ultrasound image data to identify a portion representing a region of interest is the use of an appropriately trained machine-learning algorithm. An appropriately trained machine-learning algorithm is able to process image data (or a portion thereof) to identify a portion (representing a potential regions of interest) within the image data.

In particular, a machine-learning algorithm may be trained to process ultrasound image data to identify a first portion representing a first region of interest. In some examples, a machine-learning algorithm is trained to process ultrasound image data or a first portion of the ultrasound image data to identify a second portion representing a second region of interest.

One particularly suitable machine-learning algorithm for use in identifying a portion representing a region of interest is the You Only Look Once (YOLO) algorithm described in J. Redmon and A. Farhadi. Yolov3: An incremental improvement. arXiv preprint arXiv: 1804.02767, 2018. This is a region proposal network which applies a single neural network to image data or a portion of image data to detect certain objects (i.e., portions representing regions of interest). More particularly, the neural network divides the image or portion thereof into regions and predicts portions representing potential regions of interest (e.g., in the form of bounding boxes) and probabilities for each region.

In more detail, the YOLO algorithm divides an input image or portion thereof into an S×S grid. If the center of an object (i.e., region of interest) falls into a grid cell, then that grid cell is responsible for detecting that object. Each grid cell will determine any bounding box (i.e., potential portions) and confidence scores for those boxes. These confidence scores reflect the reliability of the object model contained in the box and the accuracy of the box containing the predicted object.

Each bounding box may be defined by 5 values: x, y, w, h, and confidence. The (x, y) coordinates represent the center of the box relative to the bounds of the grid cell. The width (w) and height (h) are predicted relative to the whole image. The confidence score (confidence) of the YOLO output is a regression, which is trained to output the intersection of union (IOU) between the output portion and ground truth portion.

Using the YOLO algorithm, it is possible to identify the location of portion representing a particular region of interest within the image data, e.g., the location of the portion with the highest confidence or a confidence beyond some predetermined threshold.

The YOLO algorithm provides a resource-efficient technique (in terms of processing time and/or processing power) for identifying a portion representing a region of interest.

Of course, other suitable object or region identification techniques can be used to process image data or a portion thereof to identify the location of the first/second region of interest. Examples include any suitable segmentation or object recognition technique, such as those employing a U-net architecture or the like. Whilst the YOLO algorithm provides resource-efficient processing, other techniques may provide improved accuracy and/or robustness in identifying the region(s) of interest.

Example approaches are disclosed, for instance, by Subramanian, Kalpathi R., Dina M. Lawrence, and M. Taghi Mostafavi. "Interactive segmentation and analysis of fetal ultrasound images." Visualization in Scientific Computing'97. Springer, Vienna, 1997. 115-123; Xu, Lu, et al. "Convolutional-neural-network-based approach for segmentation of apical four-chamber view from fetal echocardiography." IEEE Access 8 (2020): 80437-80446; or Yin, Jintao, et al. "Ultrasonographic Segmentation of Fetal Lung with Deep Learning." Journal of Biosciences and Medicines 9.1 (2021): 146-153. These provide a number of examples for identification of first portions or similar representing regions of interest (e.g., the heart or lungs) within image data.

It will be appreciated that, in any above-described embodiments, any generated instance of ultrasound image/motion data may be provided to a further processor and/or output to a user interface for display. In particular, the ultrasound motion data and/or data derived therefrom may be provided for display at a user interface.

For instance, the processor may be configured to provide motion output data to a user interface or further processor, the motion output data may include data derived from the ultrasound motion data. In particular, the motion output data may comprise a graph of historic or recorded values for the ultrasound motion data, e.g., allowing an individual or processor to track or monitor fetal heart rate.

The present disclosure may make use of a machine-learning algorithm, e.g., to perform identification of the presence and/or location of a first/second portion (representing first or second regions of interest respectively) within image data or a portion thereof.

A machine-learning algorithm is any self-training algorithm that processes input data in order to produce or predict output data. Here, the input data comprises ultrasound image data (or a portion thereof) and the output data comprises a location of a portion (in the image data) representing a region of interest such as a fetal heart.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example instances of image data. The training output data entries correspond to the location of a portion of the image data, representing a region of interest such as a fetal heart.

The skilled person would be readily capable of developing a processor or ultrasound system for carrying out any herein described method. Thus, each step of a flow chart may represent a different action performed by a processor, and may be performed by a respective module of the processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as random-access memory (RAM), programmable read only memory (PROM), erasable PROM (EPROM), and electrically EPROM (EEPROM). The storage media may be encoded with one or more programs that, when executed on one or more processors and/or processors, perform the required functions. Various storage media may be fixed within a processor or processor may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable (non-transitory) medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An ultrasound monitor (120) for performing ultrasound monitoring of one or more fetuses (195, 196), the ultrasound monitor comprising:
an interface (121) for communicatively coupling with an ultrasound transducer (110); and
a processor (122) configured to be operable in a multi-data mode, when the ultrasound transducer is communicatively coupled to the interface, in which the processor is configured to:
control the ultrasound transducer to iteratively produce ultrasound image data of the one or more fetuses;
process the iteratively produced ultrasound image data to continually track the location of a first portion (310) of the ultrasound image data that represents a first region of interest of the one or more fetuses; and
use the tracked location of the first portion to control the ultrasound transducer to iteratively produce ultrasound motion data, that represents a smaller region (320) of the one or more fetuses than the ultrasound image data, that changes responsive to a motion within a second region of interest contained within the first region of interest.

2. The ultrasound monitor of claim 1, wherein the ultrasound motion data does not contain a representation of the anatomical appearance of the second region of interest.

3. The ultrasound monitor of any of claims 1 or 2, wherein the processor is configured to, when operating in the multi-data mode:
for a first iteration of producing the ultrasound motion data, process the first portion of the most recently produced ultrasound image data to identify the location of the second region of interest with respect to the ultrasound transducer; and
use the identified location of the second region of interest to control the ultrasound transducer to iteratively produce the ultrasound motion data.

4. The ultrasound monitor of any of claims 1 to 3, wherein the processor is configured to, when operating in the multi-data mode:
process the iteratively produced ultrasound image data and/or the iteratively produced ultrasound motion data to predict whether or not the second region of interest has moved by more than a predetermined amount with respect to the ultrasound transducer; and
responsive to determining that the second region of interest has moved by more than the predetermined amount, process the first portion of the most recently produced ultrasound image data to update the identified location of the second region of interest with respect to the ultrasound transducer.

5. The ultrasound monitor of claim 4, wherein the processor is configured to, when operating in the multi-data mode, responsive to determining that the second region of interest has not moved by more than the predetermined amount, maintain the location of the second region of interest with respect to the ultrasound transducer.

6. The ultrasound monitor of any of claims 1 to 5, wherein the second region of interest is smaller than the first region of interest.

7. The ultrasound monitor of any of claims 1 to 6, wherein the ultrasound motion data is M-mode ultrasound data and/or Doppler-mode ultrasound data.

8. The ultrasound monitor of any of claims 1 to 7, wherein the ultrasound motion data is pulsed-wave Doppler ultrasound data.

9. The ultrasound monitor of any of claims 1 to 8, wherein the first ultrasound image data and/or the second ultrasound image data comprises B-mode ultrasound image data.

10. The ultrasound monitor of any of claims 1 to 9, wherein the processor is further configured to, when operating in the multi-data mode,
control the ultrasound transducer to iteratively produce color or power Doppler-mode ultrasound data of the one or more fetuses.

11. The ultrasound monitor of claim 10, wherein the processor is configured to process the iteratively produced ultrasound image data and the iteratively produced color or power Doppler-mode ultrasound data to continually track the location of a first portion of the ultrasound image data that represents a first region of interest of the one or more fetuses.

12. The ultrasound monitor of any of claims 1 to 11, wherein the processor is configured, when operating in the multi-data mode to:
process the ultrasound motion data to produce a measurement for each of one or more clinical parameters of the region of interest; and/or
generate a time-varying signal that changes responsive to changes in the ultrasound motion data between each iterative production of the ultrasound motion data.

13. An ultrasound system (100) comprising:
the ultrasound monitor (120) of any of claims 1 to 12; and
the ultrasound transducer (110) communicatively coupled to the interface (121) of the ultrasound monitor.

14. A computer-implemented method (600) for controlling an ultrasound transducer for performing ultrasound monitoring of one or more fetuses, the computer-implemented method comprising:
controlling (610) the ultrasound transducer to iteratively produce ultrasound image data of the one or more fetuses;
processing (620) the iteratively produced ultrasound image data to continually track the location of a first portion of the ultrasound image data that represents a first region of interest of the one or more fetuses; and
using the tracked location of the first portion to control (630) the ultrasound transducer to iteratively produce ultrasound motion data, that represents a smaller region of the one or more fetuses than the ultrasound image data, that changes responsive to a motion within a second region of interest contained within the first region of interest.

15. A computer program product comprising computer program code which, when executed on a processor (122) communicatively coupled to an ultrasound transducer (110), cause the processor to perform the method of claim 14.
